# EUROPEAN PATENT APPLICATION

(11) **EP 3 388 155 A1**
(43) Date of publication of application: **17.10.2018**
(21) Application number: 17166562.3
(22) Date of filing: 13.04.2017
(51) Int. Cl.: B06B 1/02, B06B 1/06, A61B 8/00

(54) **ULTRASOUND TRANSDUCER PROBE WITH A FACETED DISTAL FRONT SURFACE**

(71) Applicant: Koninklijke Philips N.V., 5656 AE Eindhoven (NL)
(72) Inventor: VAN DER HORST, Arjen, 5656 AE Eindhoven (NL); LOUWERSE, Marcus Cornelis, 5656 AE Eindhoven (NL); HENNEKEN, Vincent Adrianus, 5656 AE Eindhoven (NL); WEEKAMP, Johannes Wilhelmus, 5656 AE Eindhoven (NL); KUENEN, Maarten Petrus Joseph, 5656 AE Eindhoven (NL); SHULEPOV, Sergei, 5656 AE Eindhoven (NL)
(74) Representative: de Haan, Poul Erik

(57) **Abstract**

An ultrasound transducer probe (300) for intravascular operation, comprising a faceted distal front surface (302) for ultrasound emission and reception, which has at least two mutually neighboring front surface facets (304.a, 304.b) carrying respective sub-arrays (306) of micromachined ultrasonic transducer MUT elements (308), the sub-arrays of the MUT elements together forming a faceted array of MUT elements distributed over the distal front surface.

## Description

### FIELD OF THE INVENTION

The present invention relates to an ultrasound transducer probe for intravascular operation, to an intravascular ultrasonic sensor device and to an intravascular ultrasonic sensor system.

### BACKGROUND OF THE INVENTION

For diagnosing and evaluating treatment of coronary circulatory disease ultrasound imaging and blood flow measurements using ultrasound techniques are employed.

US 2010/0268089 A1 describes a multiple electrode element capacitive micromachined ultrasonic transducer (CMUT) probe that includes a forward looking or side looking catheter device having a plurality of CMUT arrays for transmitting and receiving ultrasonic energy. The CMUT arrays are disposed on a substrate in a spaced apart arrangement and are disposed at differing locations on the substrate. The CMUT arrays each comprise a plurality of CMUT elements. CMUT transducer probes of this kind are used for medical imaging applications or as an intravascular Doppler flow sensor.

### SUMMARY OF THE INVENTION

A forward looking device carrying an ultrasound transducer probe in its tip region may in operation be not well aligned with the blood vessel. Such a misalignment of the ultrasound transducer probe during intravascular blood flow measurement may give erroneous results, which would therefore render the ultrasound measurement technique unreliable.

The inventors have recognized that for increasing reliability of ultrasound-based blood flow measurements it would be desirable to provide an ultrasound transducer probe which enables increased design variability of the ultrasound beam geometry generated by micromachined ultrasound transducer (MUT) elements.

According to a first aspect of the present invention, an ultrasound transducer probe for intravascular operation is provided. The ultrasound transducer probe comprises a faceted distal front surface (hereinafter also referred to in short as the front surface or faceted front surface) for ultrasound emission and reception, which has at least two front surface facets carrying respective sub-arrays of MUT elements, the sub-arrays of the MUT elements together forming a faceted array of MUT elements distributed over the distal front surface.

The ultrasound transducer probe of the first aspect thus has a faceted distal front surface. The individual front surface facets making up the coherent front surface carry respective sub-arrays of MUT elements. The faceted distal front surface as a whole thus provides the array of MUT elements. This advantageous front surface geometry opens up a new design space for designing the geometrical parameters of ultrasound emission from the array of MUT elements of the transducer probe. In particular, a desired width of the ultrasound beam or a desired angle of ultrasound emission provided as the superposition of the ultrasound beams radiated from every MUT element can be designed in accordance with requirements of a given application case by choosing a suitable facet design of the faceted distal front surface and of the sub-arrays of MUT elements provided on the individual front surface facets. In the following, embodiments of the ultrasound transducer probe will be described.

The MUT elements are capacitive MUT (CMUT) elements in some embodiments. In other embodiments, the MUT elements are piezoelectric MUT elements.

The individual front surface facets preferably are plane.

The faceted front surface design can be used to achieve an increased beam width of ultrasound emission in comparison with a flat front surface design. Such a wider ultrasound beam achieves a larger sample volume within the vessel, and thus increases the probability of sampling the region of the blood vessel with the highest velocity. As is known per se, the central region of a blood vessel typically exhibits the highest blood velocity amount and is used for a determination of blood flow parameters. In other cases, however, the region exhibiting the highest velocity within in the blood vessel is not the central region. For instance, in case of a curvature of the blood vessel, the highest velocities are typically closer to a wall of the blood vessel that forms an outer wall with respect to the curvature.

Some embodiments achieve such a wider ultrasound beam with a faceted distal front surface that as a whole is concave. This design of the faceted front surface allows achieving a lensing effect without requiring an actual lens on the transducer probe. A wider ultrasound beam achieved using the present embodiment therefore makes the ultrasound blood flow measurement more robust against misalignment of the sensor with respect to the vessel. Thus, the transducer probe can be built with a particularly small size and at the same time provide an ultrasound beam that is wider in comparison with a known planar design of the distal front surface. A concave front surface also makes an integration into a small hypotube form factor particularly easy and it is mechanically more robust.

In advantageous variants having a concave front surface, the front surface facets have surface vectors that extend at an angle of between 20° and 40° with respect to a central longitudinal axis extending in forward direction from the front distal front surface. The central longitudinal axis typically coincides with a central longitudinal axis of a microcatheter or guidewire that is laid to form a straight line and comprises the transducer probe at its distal end. An angle of 30° is preferred.

In some other embodiments, the front surface as a whole is convex. Also in this embodiment, the beam width of the radiated ultrasound emission can achieve higher values than in the case of a flat front surface using an otherwise identical (but flat) distribution of the MUT elements. The beam width can achieve even higher values than with a concave front surface, depending on the degree of convexity. On the other hand, the manufacture of a faceted convex front surface and the integration in ultrasonic systems is more challenging.

In some of these embodiments having a concave or convex front surface, the central longitudinal axis forms a symmetry axis in that the front surface facets are arranged in a rotational symmetry with respect to this symmetry axis.

The number of front surface facets that together form the distal front surface of the ultrasound transducer probe may be chosen according to the requirements of the beam geometry, taking into account the boundary conditions of the manufacturing process. In some embodiments, only two front surface facets are provided. In other embodiments, between three and nine front surface facets are used. Some embodiments have five front surface facets. The maximum number of facets that can be used in a given embodiment is only limited by the lateral extension of the individual MUT elements. The individual MUT elements require a respective flat surface facet.

In some embodiments, the front surface facets are formed by individual rigid carrier islands, each carrier island comprising at least one of the sub-arrays of MUT elements. Further, the carrier islands are connected to each other mechanically by a flexible electrically insulating material. This manufacturing technology allows a particularly robust and at the same time mechanically flexible design.

In case the subarrays are connected separately, they can be driven independently. However this will add complexity and/or extra connections. In other embodiments, the rigid carrier islands contain respective contact interfaces for electrically connecting the respective sub-arrays of MUT elements, and the flexible material contains electrical interconnect lines that electrically connect the contact interfaces of the different sub-arrays to each other. By connecting the sub-arrays together, the complexity is reduced, but at the cost that they cannot be driven independently.

In both cases the flexible interconnection can be just to connect the rigid islands or to also have an electrical connection.

In other embodiments, the rigid carrier islands contain respective contact interfaces for electrically connecting the respective sub-arrays of MUT elements. These embodiments further comprise a flexible interconnect bus that electrically connects the contact interfaces of the carrier islands to a rigid contact island. This rigid contact island comprises a contact interface for connection of the array of MUT elements to an external power source that drives transmission of ultrasound radiation from the MUT elements of the respective sub-array, and for connection to an external signal processing device that receives transducer signals indicative of ultrasound radiation received by the MUT elements of the respective sub-array. The rigid contact island for instance contains bondpads to enable a connection of wires. Optionally, the contact island and/or rigid carrier islands contain a signal amplification and/or signal processing unit, suitably implemented as an (optionally distributed) application specific integrated circuit (ASIC).

In other embodiments, the rigid carrier islands contain respective contact interfaces for electrically connecting the respective sub-arrays of MUT elements. In these embodiments the sub-arrays of MUT elements are connected by electrical wires to an external power source that drives transmission of ultrasound radiation from the MUT elements of the respective sub-array, and for connection to an external signal processing device that receives transducer signals indicative of ultrasound radiation received by the MUT elements of the respective sub-array.

According to a second aspect of the present invention, an intravascular ultrasonic sensor device is provided. The intravascular ultrasonic sensor device comprises a sensor body suitable for insertion into a blood vessel of a living being and an ultrasound transducer probe according to the first aspect of the invention or to any of its embodiments. The ultrasound transducer probe is preferably arranged at a distal end of the sensor body.

The intravascular ultrasonic sensor device of the second aspect thus shares the advantages of the ultrasonic transducer probe of the first aspect or of any of its embodiments.

The sensor body suitably forms a catheter, preferably a microcatheter, or a guidewire.

The sensor body provides a housing for the ultrasonic transducer probe and for signal and power transmission lines. In some embodiments of the intravascular ultrasonic sensor device the sensor body comprises a power transmission line for connection of the ultrasound transducer probe to an external power source that drives transmission of ultrasound radiation from the ultrasound transducer probe, and a signal transmission line for connection to an external signal processing device that receives transducer signals indicative of ultrasound radiation received by the MUT elements of the respective sub-array.

In some of these embodiments wherein the power transmission line is either configured for transmission of electrical power or for transmission of optical power, and the signal transmission line is either configured for transmission of electrical transducer signals or for transmission of optical transducer signals. In embodiments where either optical power or optical transducer signals are exchanged, the intravascular ultrasonic sensor preferably comprises an electro-optical power converter or an electro-optical signal converter, respectively, that is suitably located in the tip region of the intravascular ultrasonic device.

According to a third aspect of the present invention, an intravascular ultrasonic sensor system is presented. The intravascular ultrasonic sensor system comprises an intravascular ultrasonic sensor device of according to the second aspect of the present invention, a power supply device configured to provide operational power to the intravascular ultrasonic sensor device and a signal processing unit configured to receive transducer signals indicative of ultrasound echo signals received by the MUT elements and to provide a processed output signal indicative thereof.

The intravascular ultrasonic sensor system of the third aspect shares the advantages of the intravascular ultrasonic sensor device of the second aspect and of the ultrasound transducer probe of the first aspect or of any of its respective embodiments.

In some particularly advantageous embodiments, the signal processing unit is configured to determine from the transducer signals a blood flow velocity as a function of time.

In some embodiments of the third aspect, the signal processing unit of intravascular ultrasound sensor system further comprises a pre-processing unit arranged within the sensor body and configured to receive the transducer signals and to provide a pre-processed output signal indicative thereof. The pre-processing unit is preferably arranged on the rigid contact island and in some embodiments comprises an application specific integrated circuit that is configured to amplify or process the transducer signals and thus generate the pre-processed output signal.

Yet other embodiments of the intravascular ultrasound sensor system further comprise a beam control unit which is configured to control a respective phase of ultrasound emission from a number of MUT elements independently in accordance with a pre-selectable beam pattern to be emitted by the ultrasound transducer probe.

These embodiments are advantageously configured to enable an electronic steering of the ultrasound emission. The beam control unit is configured to provide power to different sets of MUT elements independently of each other. These different sets can comprise each sub-array of MUT elements, or any other combination of MUT elements, including individual MUT elements, that can then be operated independently.

It shall be understood that the ultrasound transducer probe of claim 1, the intravascular ultrasonic sensor device of claim 8, and the intravascular ultrasonic sensor system of claim 12 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Figs. 1A and 2A show a typical ultrasonic sensor device known from the prior art, as inserted into a blood vessel of a living being at two different positions and orientations.
Figs. 1B and 2B show two typical plots of the blood flow velocity as a function of time within a sample volume as resulting from such an ultrasonic measurement at the two respective different positions and orientations.
Fig. 3A shows a schematic diagram of an embodiment of an ultrasound transducer probe for intravascular operation having a concave front surface with five front surface facets.
Fig. 3B shows a schematic diagram of an embodiment of an ultrasound transducer probe for intravascular operation having a convex front surface with four front surface facets.
Fig. 4A shows ultrasound radiation patterns of a typical round transducer tip
Fig. 4B shows ultrasound radiation patterns of an ultrasound transducer probe having a concave front surface with five front surface facets.
Fig. 5 shows a diagram of another embodiment of an ultrasound transducer probe.
Fig. 6 shows an embodiment of an intravascular ultrasonic sensor system comprising an intravascular sensor device, a power source and a signal processing unit.
Fig. 7 shows another embodiment of an intravascular ultrasonic sensor system.
Fig. 8 shows another embodiment of an intravascular ultrasonic sensor system including a pressure sensor.

### DETAILED DESCRIPTION OF EMBODIMENTS

Assessing the hemodynamic significance of cardiovascular and peripheral vascular disease by intravascular flow measurement has been shown beneficial to guide treatment of atherosclerotic disease. Especially, in the coronary arteries large clinical trials have proven that decision-making based on pressure and flow measurements improves clinical outcome compared to angiography alone.

Guidewires with a Doppler ultrasound (US) sensor were developed more than two decades ago and are equipped with a single element piezoelectric ultrasound transducer based on Lead zirconate titanate (PZT). With these devices an US pulse can be sent and received. By analysis of the frequency difference between the sent and received signals, the blood velocity in a specific sampling area can be deduced as in standard ultrasound pulsed Doppler measurements.

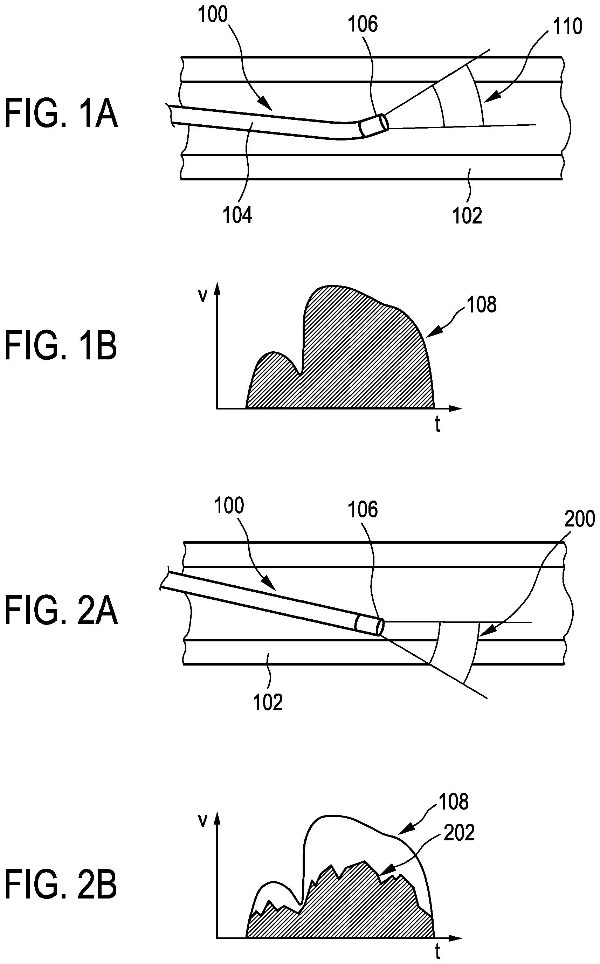

Fig. 1A shows a typical ultrasonic sensor device 100 known from the prior art, as inserted into a blood vessel 102 of a living being. The ultrasonic sensor device 100 comprises a body section 104 in the form of a guidewire and a transducer tip 106. The ultrasonic sensor device can be used for ultrasonic imaging and/or for ultrasonic Doppler measurements in determining a time dependence of blood flow velocity inside the vessel, for instance in the coronary arteries. A diagram showing a typical plot of the blood flow velocity as a function of time within a sample volume 110 as resulting from such an ultrasonic measurement is shown in Fig. 1B. The maximum velocity at each time that is measured is used as a surrogate for the flow. Typically, the maximum blood flow velocity is measured close to the center line of the vessel.

Fig. 2A shows the typical ultrasonic sensor device 100 under misalignment of the transducer probe. Considering that a location or orientation of a tip of the guide-wire can vary during a measurement, especially in coronary arteries which are located on the beating heart, this misalignment issue can make it very difficult to get a reliable peak velocity signal. Fig. 2B shows a determined maximum blood flow velocity as a function of time 202 under the measurement conditions shown in Fig. 2A compared to the maximum blood flow velocity 108 determined under the measuring conditions shown in Fig. 1A. In the former case, the sample volume 200 does not contain the regions where blood flows at a maximum flow velocity. Such a misalignment may also lead to suboptimal signal-to-noise ratio which will also difficult the determination of the maximum flow velocity.

The ultrasonic transducer probe of this invention is advantageously designed to increase the reliability of the determination of the maximum blood flow velocity by increasing the effective sample volume in which the blood flow velocity can be determined, as is will be explained in the following.

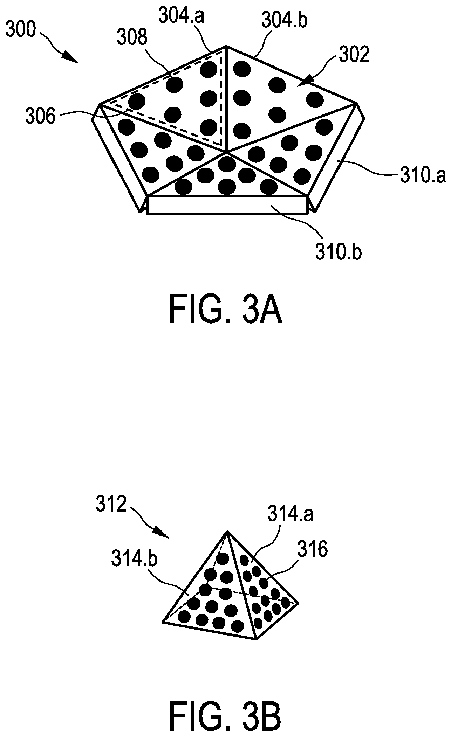

Fig. 3A shows a schematic diagram of an embodiment of an ultrasound transducer probe 300 for intravascular operation. The ultrasound transducer probe comprises a faceted distal front surface 302 for ultrasound emission and reception and comprises five neighboring front surface facets, two of which are labeled 304.a and 304.b in Fig. 3. Each of the five front surface facets carries respective sub-arrays of capacitive micromachined ultrasound transducer (CMUT) elements. For example, CMUT element 308 is an element of a sub-array 306 that is located on the front surface facet 304.a. The sub-arrays of CMUT elements thus form a faceted array of CMUT elements that are distributed over the distal front surface 302. In a variant, instead of CMUT elements, piezoelectric micromachined ultrasound transducer (PMUT) elements are used.

According to the invention, at least two of the facets are not parallel to each other. In other words, at least two facets have normal vectors at are not parallel to each other, making that that the direction of ultrasound transmission and reception of these two facets are in mutually different directions.

The front surface facets of the ultrasound transducer probe 300 of Fig. 3A are formed by individual rigid carrier islands, such as 310.a and 310.b, wherein each of the carrier islands comprise at least one of the sub-arrays of CMUT or PMUT elements. The front surface 302 of the ultrasound transducer probe presented in Fig. 3A has a concave shape as a whole.

An alternative ultrasound transducer probe is shown in Fig. 3B. The ultrasound transducer probe 312 has a distal front surface that is as a whole convex. The front surface of the ultrasound transducer probe is formed by 4 neighboring front surface facets, two of which are labeled 314.a and 314.b. Each front surface facet carries a respective sub-array of CMUT or PMUT elements 316 that form a faceted array of CMUT or PMUT elements distributed over the front surface.

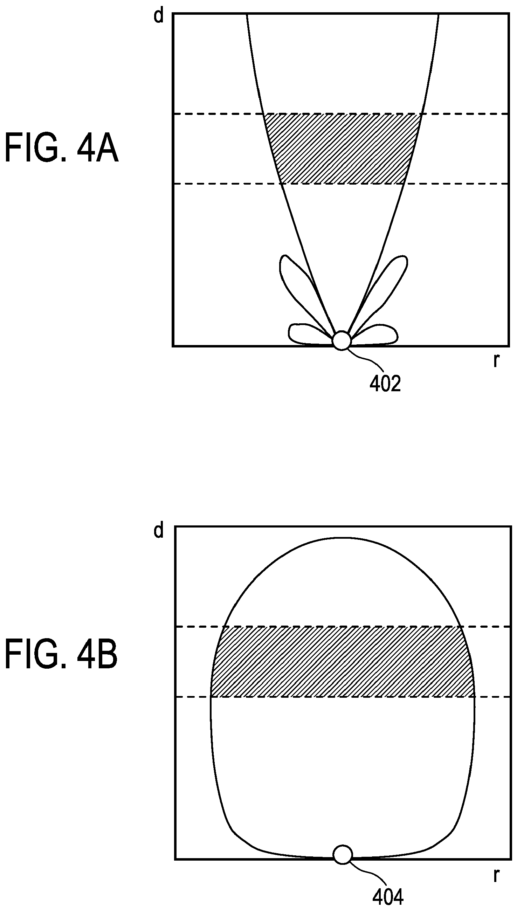

The ultrasound radiation patterns of a typical round transducer tip such as the one described with reference to Figs. 1A and 2A and of an ultrasound transducer probe such as the one of Fig. 3 are shown in Figs. 4A and 4B respectively. Fig. 4A presents a schematic diagram showing the radiation pattern at a given radiation intensity plotted versus the axial distance (d) and the radial distance (r). The radiation pattern is produced by a radiation source 402 in the form of a typical round transducer tip without any facetted front surface (e.g. transducer tip 106 of Figs. 1A and 2A). Fig. 4B presents another schematic diagram showing the radiation pattern at a given radiation intensity plotted versus the axial distance (d) and the radial distance (r). The radiation pattern is in this case produced a radiation source 404 in the form of an ultrasonic transducer probe comprising a faceted distal front surface including five front surface facets at 30° with respect to a longitudinal symmetry axis, and thus presenting a front surface that is as a whole concave. The region of interest is given by the dotted lines in both graphs. The sample volume shown in Fig. 4A as a lined region at the intersection of the region of interest and the radiation pattern has a smaller size than the sample volume shown in Fig. 4B (also the lined region at the intersection of the region of interest and the respective radiation pattern).

Therefore, the geometrical configuration of the ultrasound transducer probe having a faceted distal front surface is advantageously designed to increase a beam angle of the radiation pattern and, in turn, to increase the sample volume, thus increasing the probability of sampling a vessel region with the maximum blood flow velocity.

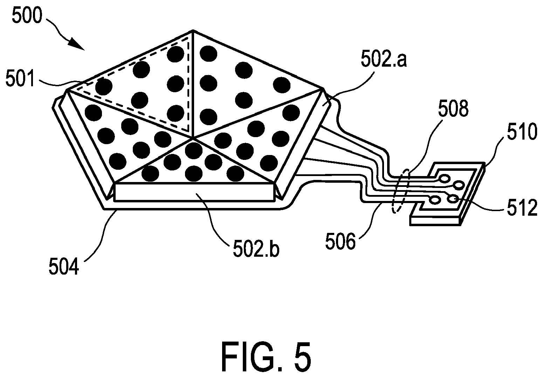

Fig. 5 shows another embodiment of an ultrasound transducer probe 500 also having 5 front surface facets, each comprising a respective sub-array 501 of CMUT elements. As before, PMUT elements could be employed in the alternative. For the purpose of simplicity, the following description will be based on the example of sub-arrays of CMUT elements. The front surface facets are formed by individual rigid carrier islands, such as for example 502.a and 502.b. All carrier islands are connected to each other mechanically by a flexible insulating material 504, such as, for example a polyimide film. The rigid carrier island contains respective contact interfaces (not shown) for electrically connecting the respective sub-arrays of CMUT elements. The flexible material 504 contains electrical interconnect lines 508 that electrically connect the contact interfaces of the different sub-arrays. The flexible material 504 also forms a flexible interconnect bus 506 that electrically connects the contact interfaces of the carrier islands to a rigid contact island 510. This rigid contact island comprises a contact interface 512 for connection of the array of CMUT elements to an external power supply (not shown) and to an external signal processing unit (not shown). The external power supply is configured to drive transmission of ultrasound radiation from the CMUT elements of the respective sub-array and the external signal processing unit is configured to receive transducer signals indicative of ultrasound radiation received by the CMUT elements of the respective sub-arrays. In these embodiments, the electrical connection between the electronics on the CMUT array side and the power source and the signal processing unit that typically include patch cables takes place on the rigid contact island, which contains the contact interface, for example, in the form of bonding pads.

The rigid carrier islands are in some ultrasound transducer probes connected with polyimide film that form hinges that enable a variation of the orientation of the single carrier islands and that include embedded electrical interconnects. These polyimide film with embedded interconnects can be advantageously manufactured using F2R (flex-to-rigid) technique. F2R is a platform that allows for the fabrication of miniature and flexible sensor assemblies for minimally invasive medical instruments such as catheters and guidewires. Devices fabricated on silicon wafers are transferred onto polyimide and partially rendered flexible by means of a two-step backside silicon deep reactive ion etching. The flexibility allows for the devices to be folded into, or wrapped around the tip of the instruments. The fabrication of the whole assembly is based on standard integrated circuit manufacturing technologies so that it is possible to scale the devices down to the dimension of the smallest guidewires.

Other ultrasonic transducer probes include rigid carrier islands that contain respective contact interfaces for electrically connecting the respective sub-arrays of CMUT elements by electrical wires to an external power source and to an external signal processing unit. These electrical contact interfaces are, in some embodiments, vias that transverse the rigid carrier island and that may be arranged at a back side or at a lateral surface of the carrier island. In these particular embodiments, the electrical connection between the sub-arrays of CMUT elements and the patch cables that are electrically connected to the power source and to the signal processing unit takes place directly at the rigid carrier islands.

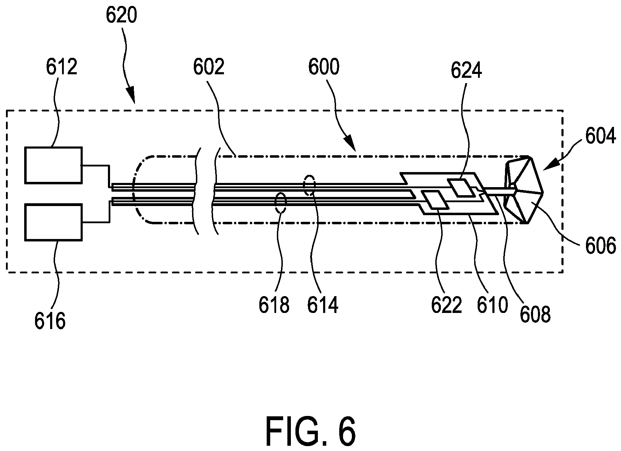

Fig. 6 shows an embodiment of an ultrasonic sensor device 600 for intravascular operation. The ultrasonic sensor device 600 comprise a sensor body 602 that is suitable for insertion into a blood vessel of a living being and an ultrasound transducer probe 604 for emission ultrasound radiation and reception of corresponding ultrasound echo radiation. The ultrasound transducer probe 604 is arranged at a distal end of the sensor body 602, which can be, for example, a catheter (particularly a microcatheter) or a guidewire. The ultrasound transducer probe 604 comprises a front surface having five front surface facets that form a concave front surface. Other ultrasound transducer probes may comprise a different number of surface facets and/or a different geometrical configuration including a convex front surface. Ultrasound transducer probes having a convex front surface produce radiation with a larger beam angle, and therefore provide a larger sample volume, but are more difficult to manufacture and integrate than ultrasound transducer probes with a concave front surface.

The CMUT faceted array of CMUT elements formed by a plurality of facets such as 606 are electrically connected to interconnect lines (not shown) that are arranged on a flexible material that forms a flexible interconnect bus 608 that connects to a rigid contact island 610. The rigid contact island is further connected to an external power source 612 by means of a power transmission line 614. The rigid contact island is also connected to an external signal processing unit 616 via a signal transmission line 618. The power source 612 drives the transmission of ultrasound radiation from the ultrasound transducer probe 604 whereas the signal processing unit receives transducer signals indicative of ultrasonic radiation received by the CMUT elements. As in previous embodiments, a PMUT faceted array of PMUT elements could be used in the alternative.

The intravascular ultrasonic sensor device 600, together with the power source 612 and the signal processing unit 616 form an exemplary embodiment of an intravascular sensor system 620.

In some intravascular sensor systems, such as 620, the signal processing unit comprises a pre-processing unit 622 arranged within the sensor body 602 and configured to receive the transducer signals and to provide a pre-processed output signal indicative thereof. The signal processing unit is advantageously arranged on the bonding stage 610 and may include an application specific integrated circuit that enables signal amplification or any other particular kind of signal processing.

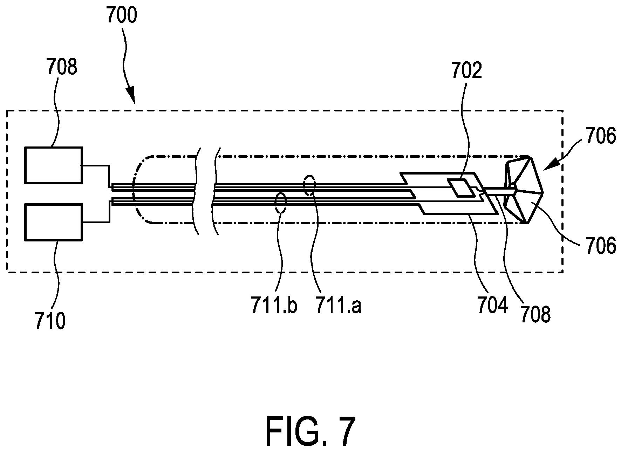

Some intravascular ultrasound sensor systems 700, such as the one depicted in Fig. 7 also comprise a beam control unit 702 arranged within the sensor body 703, preferably on the rigid contact island 704 which connects the array of MUT elements of the ultrasound transducer probe 706 to the power source 708 and the signal processing unit 710 via a power transmission line 711.a and signal transmission line 711.b. The beam control unit 702 is configured to control a respective phase of ultrasound emission from a number of MUT elements independently in accordance with a pre-selectable beam pattern to be emitted by the ultrasound transducer probe. These intravascular ultrasound sensor systems can be thus configured to control the emission of ultrasound radiation from a sub-array of MUT elements independently of each other. Some of these intravascular ultrasound sensor systems can be further configured to control the emission of ultrasound radiation for each MUT element or for a predetermined set of MUT elements independently. These intravascular ultrasound sensor systems are advantageously configured to control the emission of ultrasound radiation by controlling which of the MUT elements provides ultrasound radiation at a given time, thus enabling an electronic beam steering of the ultrasound radiation.

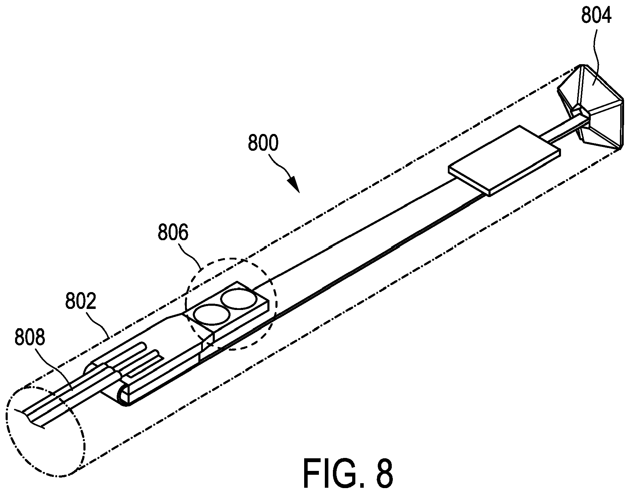

Yet other intravascular sensor systems include an intravascular ultrasonic sensor device such as the one shown in Fig. 8. The intravascular ultrasonic sensor device 800 comprises a sensor body 802 and an ultrasound transducer probe 804 comprising a faceted distal front surface, such as the one described with reference to Fig. 3. These intravascular ultrasonic sensor devices further comprise a pressure sensor 806 arranged on the sensor body and configured to provide a pressure signal indicative of an amount of blood pressure detected in the blood vessel. The pressure signal is provided also to the signal processing unit via a signal transmission line 808. These intravascular sensor systems are advantageously configured to enable the determination of both blood pressure and blood flow velocity and therefore enable an assessment of a resistance to blood flow of a given local narrowing of the vessel and of its microcirculation.

In some intravascular ultrasonic devices the power transmission line is either configured for transmission of electrical power or for transmission of optical power, and the signal transmission line is either configured for transmission of electrical transducer signals or for transmission of optical transducer signals. In cases where either optical power or optical transducer signals are exchanged, the intravascular ultrasonic sensor comprises an electro-optical power or an electro-optical signal converter respectively that is suitably located in the tip region of the intravascular ultrasonic device.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

Any reference signs in the claims should not be construed as limiting the scope.

## Claims

1. An ultrasound transducer probe (300) for intravascular operation, comprising a faceted distal front surface (302) for ultrasound emission and reception, which has at least two front surface facets (304.a, 304.b) carrying respective sub-arrays (306) of micromachined ultrasonic transducer elements, hereinafter MUT elements (308), the sub-arrays of the MUT elements together forming a faceted array of MUT elements distributed over the distal front surface.

2. The ultrasound transducer probe of claim 1, wherein the front surface (302) as a whole is concave.

3. The ultrasound transducer probe of claim 1, wherein the front surface as a whole is convex.

4. The ultrasound transducer probe of claim 1, wherein
- the front surface facets are formed by individual rigid carrier islands (502.a, 502.b), each carrier island comprising at least one of the sub-arrays of MUT elements, and wherein
- the carrier islands are connected to each other mechanically by a flexible electrically insulating material (502).

5. The ultrasound transducer probe (500) of claim 4, wherein
- the rigid carrier islands contain respective contact interfaces for electrically connecting the respective sub-arrays of MUT elements; and wherein
- the flexible material contains electrical interconnect lines (508) that electrically connect the contact interfaces of the different sub-arrays to each other.

6. The ultrasound transducer probe of claim 4, wherein
- the rigid carrier islands contain respective contact interfaces for electrically connecting the respective sub-arrays of MUT elements; and further comprising
- a flexible interconnect bus (506) that electrically connects the contact interfaces of the carrier islands to a rigid contact island (510); wherein
- the rigid contact island comprises a contact interface (512) for connection of the array of MUT elements to an external power source that drives transmission of ultrasound radiation from the MUT elements of the respective sub-array, and for connection to an external signal processing device that receives transducer signals indicative of ultrasound radiation received by the MUT elements of the respective sub-array.

7. The ultrasonic transducer probe of claim 4, wherein
- the rigid carrier islands contain respective contact interfaces for electrically connecting the respective sub-arrays of MUT elements; and wherein
- the sub-arrays of MUT elements are connected by electrical wires to an external power source that drives transmission of ultrasound radiation from the MUT elements of the respective sub-array, and for connection to an external signal processing device that receives transducer signals indicative of ultrasound radiation received by the MUT elements of the respective sub-array.

8. An intravascular ultrasonic sensor device (600) for intravascular operation, comprising;
- a sensor body (602) suitable for insertion into a blood vessel of a living being; and
- an ultrasound transducer probe (604) according to claim 1 at a distal end of the sensor body.

9. The intravascular ultrasonic sensor device of claim 8, wherein the sensor body comprises an intravascular guidewire or an intravascular catheter that comprises a power transmission line (614) for connection of the ultrasound transducer probe to an external power source (612) that drives transmission of ultrasound radiation from the ultrasound transducer probe, and a signal transmission line (618) for connection to an external signal processing device (616) that receives transducer signals indicative of ultrasound radiation received by the MUT elements of the respective sub-array.

10. The intravascular ultrasonic sensor device of claim 9, wherein the power transmission line is either configured for transmission of electrical power or for transmission of optical power, and wherein the signal transmission line is either configured for transmission of electrical transducer signals or for transmission of optical transducer signals.

11. The intravascular ultrasonic sensor device (800) of claim 8, further comprising a pressure sensor (806) arranged on the sensor body (802) and configured to provide a pressure signal indicative of an amount of blood pressure detected in the blood vessel.

12. An intravascular ultrasonic sensor system (620), comprising:
- an intravascular ultrasonic sensor device of claim 8 (600);
- a power supply device (612) configured to provide operational power to the intravascular ultrasonic sensor device; and
- a signal processing unit (616) configured to receive transducer signals indicative of ultrasound echo signals received by the MUT elements and to provide a processed output signal indicative thereof.

13. The intravascular ultrasonic sensor system of claim 12, wherein the signal processing unit (616) comprises a pre-processing unit (622) arranged within the sensor body and configured to receive the transducer signals and to provide a pre-processed output signal indicative thereof.

14. The intravascular ultrasound sensor system (700) of claim 12, further comprising a beam control unit (702), which is configured to control a respective phase of ultrasound emission from a number of MUT elements independently in accordance with a pre-selectable beam pattern to be emitted by the ultrasound transducer probe.
